# EUROPEAN PATENT APPLICATION

(11) **EP 0 838 204 A2**
(43) Date of publication of application: **29.04.1998**
(21) Application number: 97117506.2
(22) Date of filing: 09.10.1997
(51) Int. Cl.: A61F 2/38

(54) **Prosthetic knee joint with enhanced posterior stabilization**

(30) Priority: 09.10.1996 US 27771 P
(71) Applicant: Pappas, Michael J., Caldwell, N.J. 07006 (US)
(72) Inventor: Pappas, Michael J., Caldwell, N.J. 07006 (US)
(74) Representative: Müller-Boré & Partner Patentanwälte

(57) **Abstract**

The prosthetic knee joint is provided for resisting valgus-varus movements. The joint includes a femoral component having a superior bone engaging surface and an inferior bearing surface. A posterior notch extends anteriorly into the posterior end of the femoral component. The prosthetic knee joint further includes a tibial component having an inferior bone engaging surface and a superior bearing surface. A plastic bearing is disposed between the femoral and tibial components. An inferior surface of the plastic bearing is in bearing engagement with the superior surface of the tibial component. The superior surface of the plastic bearing is in articular bearing engagement with the bearing surface of the femoral component. The bearing includes a post projecting proximally from the superior surface. The post is slidably received in the posterior notch of the femoral component and resists valgus-varus moments imposed upon the joint.

## Description

### BACKGROUND OF THE INVENTION

1. Field of the Invention. The subject invention relates to a knee joint prosthesis having enhanced valgus-varus stability.

2. Description of the Prior Art. A prior art knee joint prosthesis includes a femoral component securely mounted to the distal end of a resected femur, a tibial component securely mounted to the proximal end of a resected tibia and a bearing disposed between the femoral and tibial components. The inferior face of the femoral component includes a pair of condyles. The condyles have a convexly arcuate shape, and the superior surface of the bearing has a pair of arcuate concave regions for articular bearing engagement with the condyles of the femoral component. The superior face of the tibial component may be substantially planar and is in bearing engagement with the inferior face of the bearing.

Some prior art knee joint prostheses fixedly mount the inferior surface of the bearing to the superior surface of the tibial component. Other knee joint prostheses permit either rotary movement or anterior-to-posterior sliding movement between the bearing and the tibial component. Still other prior art knee joint prostheses permit both rotary movement and anterior-to-posterior sliding movement between the bearing and the tibial component. The sliding movement of the bearing against the tibial component achieves many functional advantages described in the prior art. These functional advantages include an avoidance of dislocation in response to normal walking movement without reliance upon a fixed hinged connection. Very effective prior art knee joint prostheses incorporating certain of the structural features referred to herein are disclosed in U.S. Patent No. 4,470,158 and U.S. Patent No. 4,309,778.

Valgus is a Latin term which translates roughly as bowlegged or knock-kneed. Varus also is a Latin word that translates roughly as crooked, and is used generally to define an abnormal position of a bone of the leg or foot. Valgus-varus stability of a knee joint refers to the ability of the joint to resist lateral forces or rotary forces that would tend to urge one knee toward or away from the other. In a knee joint prosthesis, lateral forces or rotary moments that would tend to urge one knee toward or away from the other will also tend to create a dislocation particularly on one side of the prosthesis or the other.

Normal articulation of a knee joint causes the tibia to undergo rotation relative to the femur about the longitudinal axis of the tibia. Thus, the tibia articulates generally about a medial-lateral axis while simultaneously rotating about its own longitudinal axis.

A valgus-varus instability may cause one of the condyles of the femoral component to lift away from the bearing as shown in the prior art prosthesis of FIG. 14. This can lead to a phenomenon referred to as spinout subluxation during certain ranges of articulation of a prosthetic joint in a knee. Spinout subluxation is a particular problem in fixed bearing knee prostheses. More specifically, the superior surface of the bearing on a fixed bearing prosthesis tends to be less concave than the superior surface of the bearing on other prostheses. This flatter profile of the superior surface is required to permit some rotation of the tibia and the bearing affixed thereto relative to the femur. The lifting of one condyle away from the bearing due to a valgus-varus instability combined with the rotation of the tibia about its axis during certain ranges of articulation can cause one femoral condyle to spin out of its concave condyle and ride over the convex region between the two concave condyles of the bearing.

To prevent spinout subluxation some prior art fixed bearing prostheses have formed an aperture through the femoral component at a location between the two condyles. The aperture is effectively closed, and is bounded by controlling surfaces at the medial and lateral extremes and at the anterior and posterior extremes. The fixed bearing of this prior art prosthesis further includes a post extending upwardly from the superior surface and movably engaged within the aperture through the femoral component. The peripheral surfaces defining the aperture will positively limit anterior and posterior movement as well as limiting medial and lateral movement of the post in the aperture of the femoral component. Thus, the prior art prosthesis of this type provides valgus-varus stability. However, the post also is restrictive to the natural movement of the tibia relative to the femur during normal articulation. In particular, the engagement of the post in the aperture of the femoral component can significantly limit the amount of rotational movement of the tibia about its axis during normal articulation. Of course, the fixed bearing prosthesis inherently provides restrictive movement, as compared to the natural healthy knee and as compared to knee prostheses where the bearing is not fixed to the tibial component. The use of the post extending upwardly from the superior surface of the bearing and engaged in the aperture through the femoral component provides the increased valgus-varus stability at the expense of further restricting mobility in the fixed bearing prosthesis.

During normal activities, and with the prosthetic knee joint under compressive loading, the valgus-varus moments are resisted primarily and adequately by the articulating surfaces. However, there may be certain instances where under extreme forces and/or at extreme ranges of movement of the prosthetic components, additional valgus-varus stability may be desired.

Accordingly, it is an object of the subject invention to provide a prosthetic knee joint having an enhanced valgus-varus stability.

It is a further object of the subject invention to provide enhanced valgus-varus stability in a prosthetic knee joint without requiring a hinged connection.

### SUMMARY OF THE INVENTION

The subject invention is directed to a knee joint prosthesis having a femoral component and a tibial component. A bearing is disposed between the femoral and tibial components. The bearing is in rotary and/or sliding bearing engagement with the tibial component and is in articulating bearing engagement with the femoral component. The bearing and the tibial component may include means for limiting rotational and/or sliding movement therebetween. For example a post may project upwardly from the anterior portion of the superior surface of the tibial component, and may be engaged in a groove on the inferior face of the bearing. The dimensions of the groove control the amount of rotary movement. Posterior regions of each component of the prosthetic joint may include a substantially centrally disposed notch for accommodating a retained posterior cruciate ligament. The posterior notch in the femoral component may be between the condyles thereof. To resist valgus-varus moments, the bearing of the subject prosthetic joint includes a post extending proximally from a posterior region on the superior surface of the bearing and into at least a portion of the notch between the condyles of the femoral component. The post permits articular bearing movement between the femoral component and the bearing. However, the post provides resistance to lateral forces and/or valgus-varus moments, and thereby resists dislocation between the femoral component and the bearing in response to such forces and moments. The post may provide a close sliding engagement with the notch of the femoral component. However, the post may be somewhat narrower to provide a small amount of medial-lateral play between the bearing and the femoral component.

The prosthesis of the subject invention also avoids the spinout subluxation referred to above without duly resisting movement during normal articulation of the joint, as had been a problem with prior art fixed bearing prostheses. In particular, the tendency of the tibia to rotate about its axis during articulation of the joint is accommodated at the interface of the tibial component of the prosthesis and the inferior surface of the bearing of the prosthesis. Thus, unlike the prior art fixed bearing prostheses with a post extending from the bearing through an aperture in the femoral component, the subject prosthesis provides desirable mobility while still resisting valgus-varus moments.

The notch extending into the posterior face of the femoral component does not include a rear restricting surface comparable to the aperture through the femoral component of the prior art fixed bearing prosthesis. However, the prosthesis of the subject invention does provide adequate resistance to posterior dislocation. In particular, the prior art fixed bearing prosthesis with an aperture through the femoral component necessarily includes a relatively flat superior surface on the bearing to accommodate relative rotation of the bearing about the tibial axis and relative to the femur. However, rotation between the bearing and the femur about the tibial axis is not necessary in the subject prosthesis, in view of the rotatable engagement of the bearing on the tibial component. Consequently, the superior surface of the bearing can be more deeply concave and more nearly congruent with the bearing faces of the femoral condyles. This more congruent configuration of the femoral condyles and the superior surface of the bearing combined with the greater concavity of the superior surface of the bearing substantially avoids the need for a complete aperture through the femoral component and an associated posterior wall for engaging and restricting the movement of the bearing in a posterior direction.

### BRIEF DESCRIPTION OF THE DRAWINGS

FIG. 1 is a rear elevational view of the subject prosthesis and showing reaction to valgus-varus moments.

FIG. 2 is a cross-sectional view taken along line 2-2 in FIG. 1 and with a wall removed to show the bearing post.

FIG. 3 is a side elevational view similar to FIG. 2, but showing the prosthesis at 150° flexion.

FIG. 4 is a top plan view of the prosthesis shown in FIG. 3.

FIG. 5 is a front elevational view of the femoral component of the subject prosthesis.

FIG. 6 is a side elevational view of the femoral component.

FIG. 7 is a top plan view of the femoral component.

FIG. 8 is a front elevational view of the bearing of the subject prosthesis.

FIG. 9 is a side elevational view of the bearing.

FIG. 10 is a top plan view of the bearing.

FIG. 11 is a front elevational view of a tibial component in accordance with the subject invention.

FIG. 12 is a cross-sectional view of the tibial component taken along line 12-12 in FIG. 11.

FIG. 13 is a top plan view of the tibial component.

FIG. 14 is a front elevational view of a prior art prothesis showing the prior art reaction to valgus-varus moments.

### DETAILED DESCRIPTION OF THE PREFERRED EMBODIMENTS

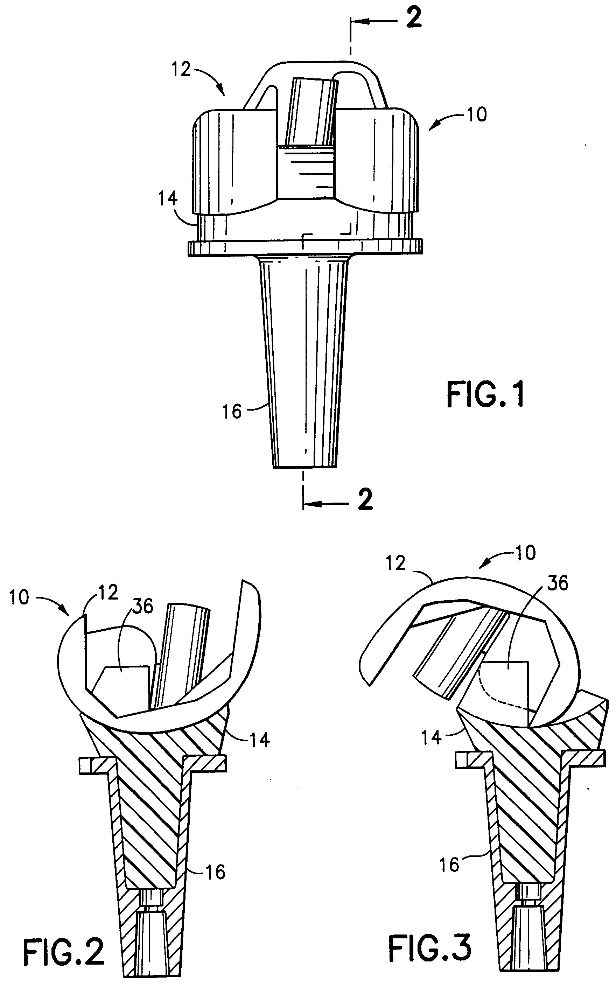

The prosthetic joint of the subject invention is identified generally by the numeral 10 in FIGS. 1-4. The prosthetic joint 10 includes a femoral component 12, a bearing 14 and a tibial component 16.

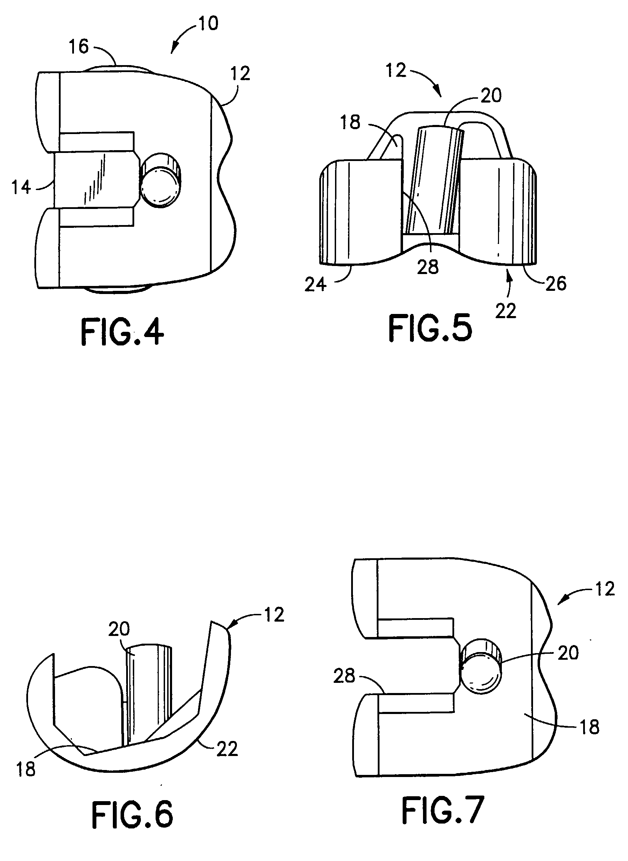

With reference to FIGS. 5-7, the femoral component 12 includes a superior mounting face 18 for mounting to the resected distal end of the femur. The mounting face 18 includes mounting post 20 for secure engagement into a cavity drilled axially into the resected distal end of the femur. Femoral component 12 further includes an inferior bearing face identified generally by the numeral 22. As shown most clearly in FIG. 5, the bearing face 22 of the femoral component 12 includes a pair of condyles 24 and 26. The femoral component 12 is further characterized by a posterior notch 28 extending from the posterior extreme of the femoral component 12 substantially to the mounting post 20. The notch 28 is partly defined by parallel medial and lateral walls which are parallel to one another.

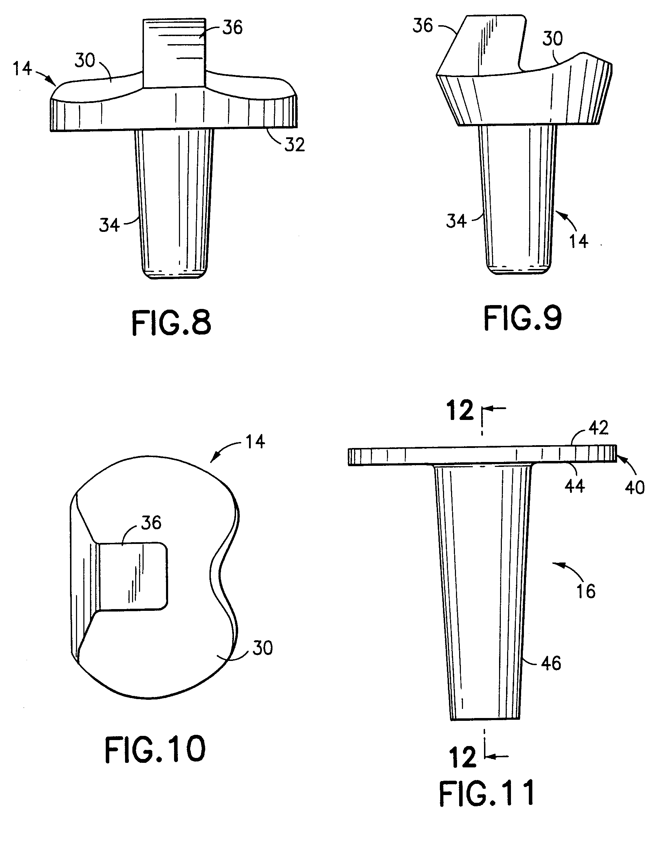

The bearing 14, as shown most clearly in FIGS. 8-10 includes a superior bearing surface 30 for articular bearing engagement with the inferior bearing face 22 of the femoral component 12. The superior bearing surface 30 is defined by a pair of concave bearing surfaces. As shown most clearly in FIGS. 2 and 3, the shapes defined by the two concave regions of the superior bearing surface 30 are substantially congruent with the convex shape defined by the condyles 24 and 26 defining the bearing face 22 of the femoral component 12. As explained further herein, this congruency provides posterior stabilization and substantially prevents posterior dislocation. The bearing 14 further includes a substantially planar inferior bearing surface 32 for rotary bearing engagement against the tibial component 16 as explained herein. A conical bearing projection 34 extends from the inferior bearing surface 32 for rotary bearing engagement in a correspondingly configured cavity in the tibial component 16 as explained herein.

The bearing 14 is further characterized by a post projecting proximally from posterior regions of the concave superior bearing surface. The post 36 has parallel planar medial and lateral faces and is dimensioned for close sliding engagement in the posterior notch 28 of the femoral component 12.

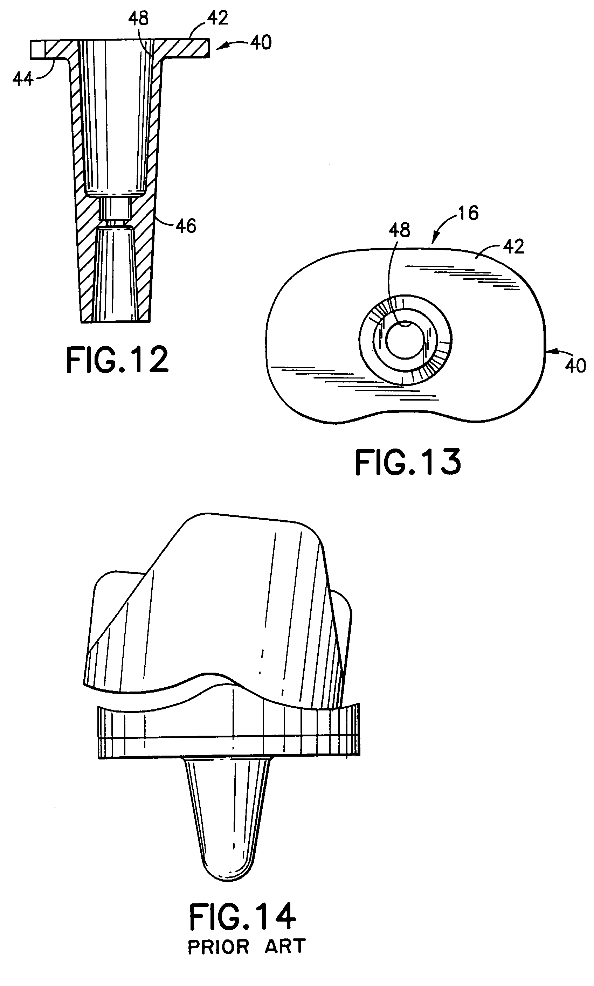

With reference to FIGS. 11-13, the tibial component 16 includes a bearing platform 40 having a superior planar bearing face 42 and an opposed inferior mounting face 44. A mounting post 46 projects distally from the inferior face 44 for engagement in the cavity drilled into the proximal end of the resected tibia. The superior bearing face 42 of the tibial platform 40 includes a cavity 48 extending axially into the mounting post 46. The cavity 48 is dimensioned to rotatably receive the conical bearing projection 34 of the bearing 14.

The components of the prosthesis 10 are assembled as shown in FIGS. 1-4. In the assembled condition, the conical bearing projection 34 of the bearing 14 is rotatably engaged in the conical cavity 48 of the tibial component 16. Thus, the inferior bearing face 32 of the bearing 14 is in rotary bearing relationship with the superior face 42 of the tibial component 16. The inferior bearing surface 22 of the femoral component 12 is in articular bearing engagement with the concave superior bearing surface 30 of the bearing 14. Additionally, the condyles 24 and 26 defining the inferior bearing surface 22 of the femoral component 12 are substantially congruent with the concave superior bearing surface 30 of the bearing 14 as shown in FIGS. 2 and 3 and as described above. The post 36 is slidably received in the posterior notch 28 of the femoral component 12. As illustrated graphically in FIG. 1, the post 36 resists valgus-varus moments imposed upon the prosthetic component. As shown in FIGS. 2 and 3, the engagement of the post 36 in the notch 28 is effective for resisting valgus-varus moments for virtually all ranges of movement of the prosthetic component. The substantial congruency of the superior bearing face 32 of the bearing 14 with the condyles 24 and 26 of the inferior bearing face 22 of the femoral component 12 substantially ensures posterior stabilization despite the fully opened posterior end of the notch 28 in the femoral component 12. Thus, valgus-varus stabilization is provided without adversely affecting mobility, and specifically while providing rotation of the bearing on the tibial component.

While the invention has been described with respect to a preferred embodiment, it is apparent that various changes can be made without departing from the scope of the invention as defined by the appended claims.

## Claims

1. A knee joint prosthesis comprising:
a femoral component having a superior bone engaging face for secure engagement to a femur and having an inferior articular bearing surface, a posterior notch projecting anteriorly into a posterior face of the femoral component;
a tibial component having an inferior bone engaging face for engagement with a tibia and a substantially planar superior bearing face; and
a bearing disposed between the femoral and tibial components, the bearing having an inferior face in bearing engagement with the superior face of the tibial component, said bearing further having a superior bearing face in articular bearing engagement with the inferior bearing surface of the femoral component, said bearing further including a post projecting proximally from the superior face thereof and disposed in the posterior notch of the femoral component for resisting valgus-varus moments imposed upon the joint.

2. The knee joint prosthesis of claim 1, wherein the inferior face of the bearing is movably disposed on the tibial component.

3. The knee joint prosthesis of claims 1 or 2, wherein the posterior notch of the femoral component includes substantially parallel medial and lateral side walls in facing relationship to one another, said post of said bearing including substantially parallel medial and lateral surfaces in opposed facing relationship to the respective medial and lateral side walls of the posterior notch in the femoral component.

4. The knee joint prosthesis of claim 3, wherein the post of the bearing is dimensioned such that the medial and lateral surfaces thereof are disposed substantially in sliding relationship with the respective medial and lateral side walls of the posterior notch in the femoral component.

5. The knee joint prosthesis of any claims 1-4, wherein the inferior articular bearing surface of the femoral component comprises a pair of convex condyles, and wherein the superior bearing face of the bearing comprises a pair of concave recesses substantially congruent with the condyles of the femoral component.

6. The knee joint prosthesis of any of claims 1-5, wherein the tibial component includes a conically generated recess extending into the superior bearing face thereof, and wherein the bearing includes a conically generated projection extending from the inferior face thereof and rotatably disposed in the recess of the tibial component, such that the inferior face of the bearing is in rotary bearing engagement with the superior bearing face of the tibial component.

7. The knee joint prosthesis of claim 6, further comprising means for limiting movement of the bearing relative to the tibial component.

8. The knee joint prosthesis of any of claims 1-7, wherein the femoral component and the tibial component are formed from a metallic material and wherein the bearing is formed from a non-metallic material.
